# EUROPÄISCHE PATENTANMELDUNG

(11) **EP 2 705 757 A1**
(43) Veröffentlichungstag der Anmeldung: **12.03.2014**
(21) Anmeldenummer: 13005082.6
(22) Anmeldetag: 04.10.2007
(51) Int. Cl.: A23C 21/02, C12P 7/06, A23C 9/144

(54) **Verfahren zur Aufarbeitung von Melasse aus Molke**

(62) Teilanmeldung aus: 07019471.7
(71) Anmelder: Molkerei Alois Müller GmbH & Co. KG, 86850 Aretsried (DE)
(72) Erfinder: Cloidt, Roland, D-01454 Radeberg (DE); Lehmann, Hanno, D-59846 Sundern (DE)
(74) Vertreter: Leinweber & Zimmermann

(57) **Zusammenfassung**

Die Erfindung betrifft ein Verfahren zur Aufarbeitung von bei der Laktosegewinnung aus Molkenpermeat zurückbleibender Melasse. Dabei wird aus der Melasse eine mineralisch abgereicherte Komponente abgetrennt, aufkonzentriert, kristallisiert und eine ausgefällte kristalline Phase abgetrennt.

## Beschreibung

Die Erfindung bezieht sich auf ein Verfahren zur Aufarbeitung von Melasse. Letztere fällt insbesondere bei der Aufarbeitung von Molke an, bei der die Molke zunächst durch ein Membranverfahren in eine proteinangereicherte Retentatkomponente und eine laktoseangereicherte Permeatkomponente getrennt wird. Aus letzterer wird nach Eindampfen und Kristallisieren durch mechanische Trennverfahren Laktose abgetrennt, wobei die Melasse als unverwertete Komponente zurückbleibt und entsorgt werden muß.

Der Erfindung liegt die Aufgabe zugrunde, ein Verfahren zur Aufarbeitung von Melasse zu schaffen, durch das wertvolle Inhaltsstoffe der Melasse gewonnen werden können.

Erfindungsgemäß wird diese Aufgabe durch ein Verfahren gelöst, das die folgenden Verfahrensschritte aufweist:
Isolieren einer mineralisch abgereicherten Komponente durch Abtrennen von Mineralien aus der Melasse,
Aufkonzentrieren der mineralisch abgereicherten Komponente durch Wasserentzug
Kristallisieren der aufkonzentrierten Komponente
Abtrennen der durch das Kristallisieren ausgefällten kristallinen Phase von der flüssigen Phase
Waschen der abgetrennten kristallinen Phase mit einer Waschflüssigkeit.

Die dem Verfahren aus der Molkeverarbeitung zugeführte Melasse weist einen Trockenmassegehalt von beispielsweise 18 bis 35 % auf. Der verringerte Mineralgehalt der durch das Abtrennen von Mineralien isolierten, mineralisch abgereicherten Komponente ist für deren nachfolgende Kristallisation vorteilhaft.

Für die Mineralabtrennung im technischen Maßstab eignen sich die Elektrodialyse oder die Chromatographie, mit der es gelingt, 50 bis 80 %, insbesondere 60 bis 65 %, der in der Melasse befindlichen Mineralien abzutrennen. Für diese Verfahren ist es dabei vorteilhaft, den Trockenmassegehalt der dem Verfahren zugeführten Melasse auf z. B. 10 % einzustellen. Dies kann dadurch geschehen, daß der dem Verfahren zugeführten Melasse Brüden zugesetzt werden, die in nachfolgenden Verfahrensschritten entstehen. Dadurch kann die Wasserbilanz des Gesamtprozesses ausgeglichen werden, so daß kein zusätzliches Frischwasser zugeführt werden muß.

Die durch die Elektrodialyse/Chromatographie isolierte, mineralisch abgereicherte Komponente weist beispielsweise einen Trockenmassegehalt von 20 % auf. In der Trockenmasse beträgt der Aschegehalt beispielsweise 8 bis 9 % sowie der Gehalt an Glucose und Milchsäure 2 bis 5 %.

Durch das Aufkonzentrieren der mineralisch abgereicherten Komponente, das insbesondere durch Eindampfen erfolgt, wird der Trockenmassegehalt erhöht, beispielsweise auf 60 bis 70 %.

Das Kristallisieren der solchermaßen aufkonzentrierten Komponente erfolgt vorzugsweise in einem Kristallisationstank über eine Zeitdauer von beispielsweise 25 bis 35 Stunden.

Die Suspension, einschließlich der ausgefällten kristallinen Phase, kann dann entweder entsprechend der Patentanmeldung 06 012 916.0-2114 - "Verfahren zur Herstellung von Permeatpulver" - behandelt werden oder entsprechend folgendem Prozeßablauf:

Die Suspension einschließlich der kristallinen Phase wird in einer mehrstufigen, vorzugsweise einer zwei- bis vierstufigen, Dekantier- und Waschstraße von allen unerwünschten Inhaltsstoffen befreit. In der solchermaßen abgereicherten Komponente beträgt der Gehalt an Glucose und Milchsäure, die für eine Trocknung ungünstig sind, in der Trockenmasse beispielsweise weniger als 1 %, vorzugsweise weniger als 0,2 %. Somit kann diese abgereicherte Komponente wirkungsvoll einem Trocknungsvorgang unterzogen werden, dessen Endprodukt ein Laktosepulver ist.

Mit besonderem Vorteil wird die von der kristallinen Phase getrennte flüssige Phase des Kristallisationsprozesses und/oder die die abgetrennte Glucose und Milchsäure enthaltende Komponente und/oder die die abgetrennten Mineralien enthaltende Komponente zu Ethanol weiterverarbeitet. Demnach besteht eine vorteilhafte Ausführungsform darin, daß eine von mindestens einem Teil der flüssigen Phase des Kristallisationsprozesses gebildete Komponente zu Ethanol weiterverarbeitet wird. Insbesondere kann vorgesehen sein, daß die nach Abtrennung der an Glucose und Milchsäure abgereicherten Komponente verbleibende Komponente zu Ethanol weiterverarbeitet wird, oder daß die nach Isolieren der mineralisch abgereicherten Komponente verbleibende Komponente zu Ethanol weiterverarbeitet wird.

In diesem Zusammenhang ist es zweckmäßig, daß die zu Ethanol zu verarbeitende Komponente durch Zusatz von Hefe fermentiert, eine durch die Fermentierung gebildete alkoholische Komponente von der Hefe mechanisch abgetrennt und von der abgetrennten alkoholischen Komponente ein Alkoholanteil thermisch abgetrennt wird. Dabei kann vorgesehen sein, daß die mechanische Abtrennung durch einen Separator erfolgt. Weiter kann vorgesehen sein, daß die thermische Abtrennung des Alkoholanteils durch Destillieren erfolgt oder daß die thermische Abtrennung des Alkoholanteils durch Rektifizieren erfolgt.

Außerdem kann vorgesehen sein, daß die Hefe die Gattung Kluyveromyces enthält. Ferner ist zweckmäßigerweise vorgesehen, daß ein Teil der mechanisch abgetrennten Hefe zur Fermentierung zurückgeführt wird.

In der folgenden Beschreibung wird ein Ausführungsbeispiel der Erfindung anhand der Zeichnung näher erläutert. Hierin zeigen:
- Fig. 1: ein Ablaufschema eines Gesamtprozesses, und
- Fig. 2: Einzelheiten eines der Ethanolherstellung dienenden Zweiges des Gesamtprozesses von Fig. 1.

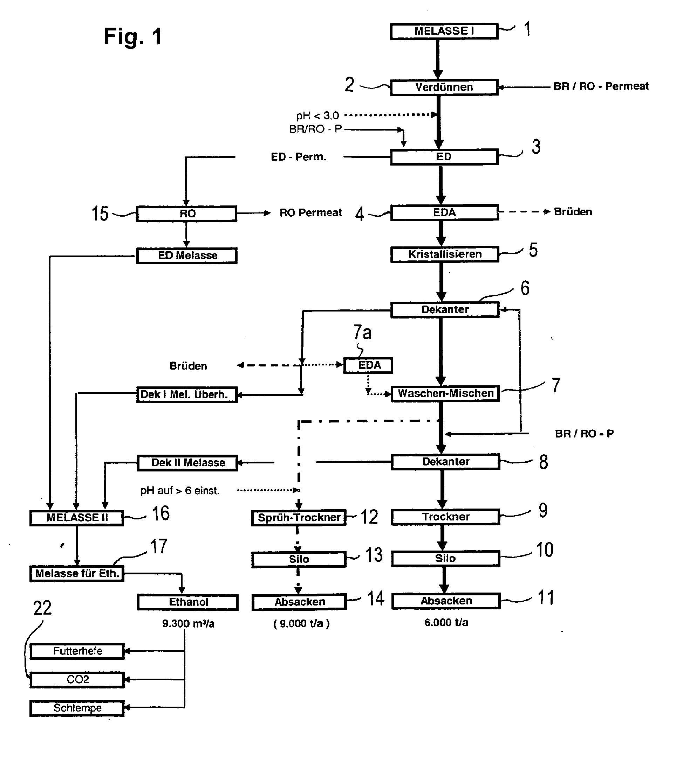

In dem Ablaufschema von Fig. 1 symbolisiert ein Block 1 die eingangsseitige Zuführung von Melasse zum Gesamtprozeß, wobei diese Melasse am Ende eines Laktoseprozesses anfällt, bei dem Molke zuerst eine proteinangereicherte Retentatkomponente entzogen und von der verbleibenden laktosereichen Permeatkomponente durch Eindampfen und Kristallisieren Laktose abgetrennt wird. In einer im Verfahrensablauf folgenden Stufe 2 wird die bei 1 zugeführte Melasse, die einen Trockenmassegehalt von etwa 25 % aufweisen kann, durch Verdünnen auf einen Trockenmassegehalt von 10 % oder kleiner standardisiert. Als Verdünnungsmittel für diese Reduzierung des Trockenmassegehaltes dienen Brüden und/oder ein Permeat aus reverser Osmose, die in noch zu erläuternden nachgeschalteten Prozeßstufen anfallen.

Der pH-Wert der solchermaßen standardisierten Melasse kann durch Zugabe von Hilfsstoffen, beispielsweise Salzsäure, auf einen pH-Wert zwischen 2 und 6 eingestellt und von der ggf. eingestellten Melasse in einer auf die Stufe 2 folgenden Stufe 3 durch Elektrodialyse ein wesentlicher Anteil der in der standardisierten Melasse enthaltenen Mineralien, beispielsweise 60 bis 65 %, abgetrennt werden. Es ist geraten, der Elektrodialyse eine mechanische Klärung, vorzugsweise einen Separator oder eine Mikrofiltration, vorzuschalten, um Partikel zurückzuhalten, die die Funktion der Elektrodialyse negativ beeinflussen würden. Die nach der Elektrodialyse verbleibende, mineralisch abgereicherte Komponente, deren Trockenmassegehalt beispielsweise etwa 20 % beträgt, wird in einer auf die Elektrodialysestufe 3 folgenden Eindampferstufe 4 auf eine Konzentration von beispielsweise 65 % Trockenmasse eingedampft und anschließend in einem Kristallisationstank 5 über beispielsweise 25 bis 35 Stunden kristallisiert.

In der dem Kristallisationstank 5 nachgeschalteten mehrstufigen Dekantier- und Waschstraße (Stufen 6, 7 und 8) werden die für die Trocknung unerwünschten Gehalte an Mineralien, Proteinen, Glucose und Milchsäure weitestgehend abgetrennt. Die abgereicherte Komponente aus dem Dekanter 8, deren Gehalt an unerwünschten Inhaltsstoffen beispielsweise kleiner als 1 %, vorzugsweise in Summe kleiner als 0,5 % ist, wird in einer dem Dekanter 8 nachgeschalteten Trocknungsstufe 9 getrocknet, wodurch als Endprodukt ein Laktosepulver entsteht, das in einem Silo 10 zwischengelagert und an einem Ausgang 11 des Silos 10 abgesackt werden kann.

Vorzugsweise sind die Prozesse so zu gestalten, daß in Abhängigkeit vom Markt Permeatpulver oder das höherwertige Laktosepulver hergestellt werden kann. Hierzu kann das in der Waschmischstufe 7 erhaltene Gemisch ganz oder teilweise nach einer pH-Wert-Einstellung auf 6 oder höher in einem Sprühtrockner 12 getrocknet werden, wodurch ein Permeatpulver entsteht, das in einem Silo 13 zwischengelagert und an einem Ausgang 14 des Silos 13 abgesackt werden kann.

Die in den Eindampferstufen 4 und 7a entstehenden Brüden werden zu der Verdünnungsstufe 2, der Elektrodialysestufe 3 sowie den Dekanterstufen 6 und 8 zurückgeführt. Dadurch kann der in den Stufen 1 bis 8 ablaufende Prozeß ohne zusätzliche Frischwasserzufuhr betrieben werden.

Die in der Elektrodialysestufe 3 bei der Gewinnung der mineralisch abgereicherten Komponente verbleibende Komponente wird in einer Stufe 15 durch reverse Osmose aufkonzentriert und das aufkonzentrierte Retentat einem Ethanolgewinnungsprozeß zugeführt. Das Permeat der Stufe 15 wird für einen Bilanzausgleich des Gesamtprozesses zu der Verdünnungsstufe 2, der Elektrodialysestufe 3 und den Dekanterstufen 6 und 8 zurückgeführt.

Dem Ethanolgewinnungsprozeß wird ferner der nicht für die Wasch-Mischstufe 7 verbrauchte Anteil der flüssigen Phase aus dem Dekanter 6 zugeführt. Dieser Anteil beträgt 70 bis 90 %, insbesondere 85 %, und führt insbesondere amorphe Laktose und Galaktose aus dem in den Stufen 1 bis 8 ablaufenden Prozeß.

Weiterhin wird dem Ethanolgewinnungsprozeß die in dem Dekanter 8 abgetrennte flüssige Phase zugeführt. Die Zusammenfassung der drei Teilströme aus der Elektrodialysestufe 3, der Dekanterstufe 6 und der Dekanterstufe 8 zu einem für die Ethanolgewinnung verwendeten Gesamtstrom ist in Fig. 1 durch die Blöcke 16 und 17 veranschaulicht.

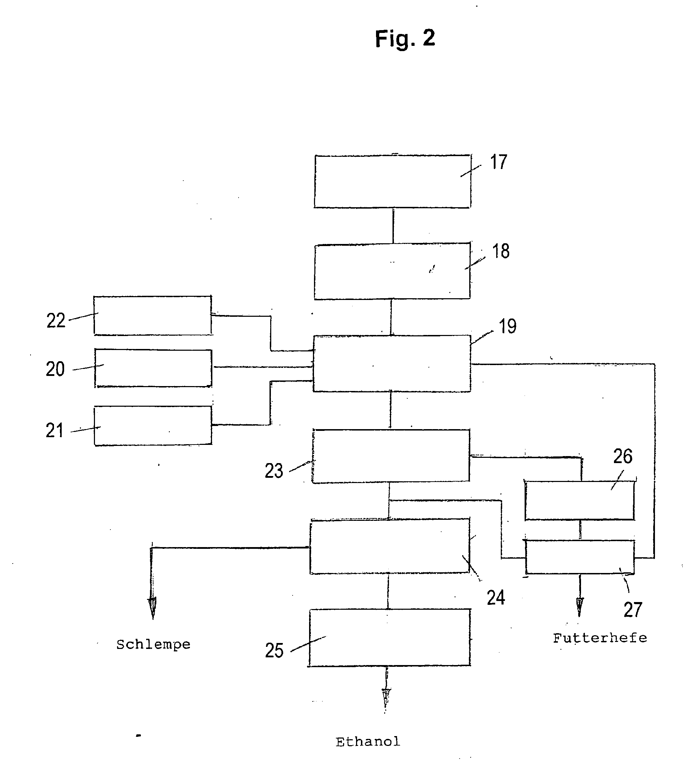

Gemäß Fig. 2 wird der in dem Block 17, bei dem es sich um einen Tank handeln kann, anfallende Gesamtstrom einem Erhitzer 18 zugeführt, in dem eine Erhitzung auf beispielsweise 85° zur Keimabtötung stattfindet. In einem dem Erhitzer 18 nachgeschalteten Fermentationstank 19 wird aus einem Kulturtank 20 Hefe zugesetzt und die Fermentation bei einer Fermentationstemperatur im Bereich vom 25 bis 35° C durchgeführt. Es werden vorzugsweise Hefen der Gattung Kluyveromyces verwendet. Die Einstellung eines geeigneten pH-Wertes, beispielsweise von 4 bis 6, erfolgt durch Zufuhr von Lauge oder Säure aus einem Vorlagebehälter 21. Das bei der Fermentation entstehende CO₂ kann in einer CO₂ Verflüssigungsanlage 22 aufgefangen werden.

Nach abgeschlossener Fermentierung wird das fermentierte Substrat in einem Separator 23 in ein Hefenkonzentrat und ein hefefreies Fermentat getrennt. Aus letzterem wird in einer Stufe 24 Rohalkohol durch Destillieren, Rektifizieren erzeugt. Dieser Alkohol mit bis zu 96 % wird in einer nachgeschalteten Dehydrationsstufe 25 beispielsweise bis auf eine Reinheit von mindestens 99,8 % entwässert, wodurch handelsfähiges Ethanol erhalten wird.

Das in dem Separator 23 abgetrennte Hefenkonzentrat wird in einer Wascheinrichtung 26 mit Wasser versetzt und anschließend in einem zweiten Separator 27 aufkonzentriert. Die in dem Separator 27 abgetrennte Klarphase wird dem der Stufe 24 zulaufenden Fermentat aus dem Separator 23 beigemischt, um enthaltene Restalkohole zurückzugewinnen. Das in dem Düsenseparator 27 abgetrennte Hefenkonzentrat wird bis zu 50 % in den Fermentationstank 19 zurückgeführt. Das nicht zurückgeführte Hefenkonzentrat wird als Futterhefe entnommen. Die beim Destillieren/Rektifizieren in der Stufe 24 anfallende Schlempe wird entnommen und kann zur weiteren Verwendung durch Eindampfen auf 20 bis 30 % Trockenmasse aufkonzentriert werden.

In Fig. 1 sind beispielhaft Jahresmengen für eine Prozeßführung im industriellen Maßstab angegeben. Danach werden ausgehend von einer jährlichen Zuführung von 29.986 t Melasse jährlich 6.000 t Laktosepulver, alternativ 9.000 t Permeatpulver und 9.300 m³ Ethanol gewonnen.

## Patentansprüche

1. Verfahren zur Aufarbeitung von Melasse, die aus einem Molkeaufbereitungsverfahren entnommen wird, bei dem der Molke zuerst Eiweiß durch ein Membrantrennverfahren und anschließend Laktose durch ein Kristallisationsverfahren entzogen wird, dessen Mutterlauge die Melasse liefert und die einen Trockenmassegehalt zwischen 18 und 35 % aufweist, mit den Verfahrensschritten:
Einstellen des Trockenmassegehalts der dem Verfahren zugeführten Melasse auf 10 % oder weniger,
Isolieren einer mineralisch abgereicherten Komponente durch Abtrennen von Mineralien aus der Melasse,
Aufkonzentrieren der mineralisch abgereicherten Komponente durch Wasserentzug durch Eindampfen,
Kristallisieren der aufkonzentrierten Komponente,
Abtrennen der durch das Kristallisieren ausgefällten kristallinen Phase von der flüssigen Phase,
Waschen der abgetrennten kristallinen Phase mit einer Waschflüssigkeit,
**dadurch gekennzeichnet, daß** das Abtrennen von Mineralien aus der Melasse durch Elektrodialyse und die Einstellung des Trockenmassegehalts durch Zugabe von in dem Eindampfschritt entstehenden Brüden erfolgt.

2. Verfahren nach Anspruch 1, **dadurch gekennzeichnet, daß** das Kristallisieren in einem Kristallisationstank erfolgt.

3. Verfahren nach einem der Ansprüche 1 bis 2, **dadurch gekennzeichnet, daß** das Abtrennen der kristallinen Phase in einem Dekanter erfolgt.

4. Verfahren nach einem der Ansprüche 1 bis 3, **dadurch gekennzeichnet, daß** die Waschflüssigkeit aus der flüssigen Phase des Kristallisationsprozesses entnommen wird.

5. Verfahren nach Anspruch 4, **dadurch gekennzeichnet, daß** die entnommene flüssige Phase zur Bildung der Waschflüssigkeit aufkonzentriert wird.

6. Verfahren nach Anspruch 5, **dadurch gekennzeichnet, daß** das Aufkonzentrieren der entnommenen flüssigen Phase durch Eindampfen erfolgt.

7. Verfahren nach einem der Ansprüche 1 bis 6, **dadurch gekennzeichnet, daß** die aufkonzentrierte Komponente einen Trockenmassegehalt zwischen 60 und 70 % aufweist.

8. Verfahren nach einem der Ansprüche 1 bis 7, **dadurch gekennzeichnet, daß** durch die Abtrennung zwischen 60 und 65 % der in der zugeführten Melasse enthaltenen Mineralien abgetrennt werden.

9. Verfahren nach einem der Ansprüche 1 bis 8, **dadurch gekennzeichnet, daß** das Kristallisieren über eine Zeitdauer von 25 bis 35 Stunden ausgeführt wird.

10. Verfahren nach einem der Ansprüche 1 bis 9, **dadurch gekennzeichnet, daß** für das Isolieren der mineralisch abgereicherten Komponente der pH-Wert auf kleiner 6,0 eingestellt wird.

11. Verfahren nach einem der Ansprüche 1 bis 10, **dadurch gekennzeichnet, daß** aus der gewaschenen kristallinen Phase durch Abtrennen eine an Glucose und Milchsäure abgereicherte Komponente gewonnen wird.

12. Verfahren nach Anspruch 11, **dadurch gekennzeichnet, daß** das Abtrennen in einem Dekanter erfolgt.

13. Verfahren nach Anspruch 11 oder 12, **dadurch gekennzeichnet, daß** die an Glucose und Milchsäure abgereicherte Komponente getrocknet wird.

14. Verfahren nach einem der Ansprüche 1 bis 10, **dadurch gekennzeichnet, daß** die gewaschene kristalline Phase sprühgetrocknet wird.

15. Verfahren nach Anspruch 14, **dadurch gekennzeichnet, daß** der pH-Wert der gewaschenen kristallinen Phase auf 6 oder höher eingestellt wird.

16. Verfahren nach einem der Ansprüche 1 bis 15, **dadurch gekennzeichnet, daß** eine von mindestens einem Teil der flüssigen Phase des Kristallisationsprozesses gebildete Komponente zu Ethanol weiterverarbeitet wird.
